# EUROPEAN PATENT SPECIFICATION

(11) **EP 1 343 009 B1**
(45) Date of publication and mention of the grant of the patent: **25.02.2009**
(21) Application number: 01270772.5
(22) Date of filing: 11.12.2001
(51) Int. Cl.: G01N 33/543, G01N 33/531

(54) **IMMUNOLOGICAL ASSAY METHOD**
IMMUNOLOGISCHES VERFAHREN
PROCEDE D'ANALYSE IMMUNOLOGIQUE

(30) Priority: 11.12.2000 JP 2000376221
(43) Date of publication of application: 10.09.2003
(73) Proprietor: Mitsubishi Kagaku Iatron, Inc., Tokyo 162-0812 (JP)
(72) Inventor: NAKAHARA, Kunihiko, c/o Mitsubishi Kagaku Iatron, Inc., Tokyo 162-0812 (JP); SAWAI, Tokio, c/o Mitsubishi Kagaku Iatron, Inc., Tokyo 162-0812 (JP)
(74) Representative: Vossius & Partner
(86) International application number: PCT/JP2001/010855
(87) International publication number: WO 2002/048711

(56) References cited:
- EP-A- 0 302 715
- EP-A- 0 464 554
- WO-A-87/04794
- JP-A- 9 304 383
- JP-A- 9 506 172
- JP-A- 11 290 097
- PATENT ABSTRACTS OF JAPAN vol. 017, no. 265 (P-1542), 24 May 1993 (1993-05-24) & JP 05 005739 A (NIPPON TELEGR & TELEPH CORP), 14 January 1993 (1993-01-14)

## Description

### TECHNICAL FIELD

The present invention relates to an immunological analyzing method. More particular, the present invention relates to an analyzing method making use of an aggregation reaction caused by an immunological reaction. The term "analyzing" or "analysis" as used herein includes a measurement to quantitatively or semi-quantitatively determine an amount of a substance to be analyzed, and a detection to judge a presence or absence of a substance to be analyzed.

### BACKGROUND ART

As a method for quantitatively analyzing a trace component in a liquid taken from a living body, an immunological measuring method involvin the use of an antibody or antigen against the substance to be analyzed is often used. As the immunological measuring method, a nepherometric immunoassay and a turbidimetric immunoassay in which an immunocomplex formed by the antigen-antibody reaction is optically measured, and a radioimmunoassay or an enzyme immunoassay in which a radioisotope or an enzyme is used as a label are carried out. An automated analyzer able to handle many samples in a short time is now widely used, and a high-sensitivity is desired. Accordingly a latex aggregation method making use of the reaction with a latex particle carrying an antibody (or an antigen) is widely used. In the latex aggregation method, a substance to be analyzed is measured by detecting the degree of aggregation of latex particles, which are formed by the reaction between the antibody (or an antigen) binding to the latex particle and the substance to be analyzed. As the method for detecting the degree of the aggregation, there may be mentioned, for example, a method in which the aggregation is visually observed, or a method in which a scattered light or a transmitted light caused by irradiating light to a reaction liquid is measured. The optical analyzing method is used to quantitatively determine an antigen or antibody in a sample.

WO-A-87/01791, EP-A-0 464 554 and EP-A-0 302 715 disclose compositions of matter comprising a biotin-conjugate antigen/antibody and avidin-conjugated latex particles.

However, to carry out the latex aggregation method, it is necessary to prepare a latex particle carrying an antibody (or antigen) against the substance to be analyzed. As this method, there may be mentioned, for example, a method of immobilization in which the antibody (or antigen) is bound to the latex particle by mixing, or a chemically binding method in which the antibody (or antigen) is covalently bound to the latex particle. These procedures are complicated, and have proved to be one of the problems in the latex aggregation method. Another and major problem in the latex aggregation method is self-aggregation of the latex particles. More particularly, when the antibody (or antigen) is carried on the latex particle, the balance of an electric double layer, which maintains the dispersibility of the latex particle per se in a solution, is disturbed. As a result, the self-aggregation of the latex particles occurs independently of the antigen-antibody reaction. This self-aggregation is the cause of an the inaccurate measurement, and an increase or decrease of sensitivity with the lapse of time may be sometimes observed, because a storage stability of the latex particle per se is lowered.

Further, when the antibody (or antigen) against the substance to be analyzed is carried on the latex particle, the antibody (or antigen) as a protein may be sometimes inactivated by adsorption of the antibody (or antigen) to the surface of the latex particle. If such an' inactivation occurs, a change occurs in the reactivity of the antibody (or antigen) against the substance to be analyzed. As a result, the desired immunological reaction does not occur, or another reaction occurs, and thus the reliability of measurement is significantly lowered.

Until now, to resolve the above problems, several methods in which the antibody (or antigen) can be easily carried on the latex particle and the self-aggregation can be inhibited have been examined, by improving a component contained in the latex particle or a process for manufacturing the latex particle. However, although this approach is beneficial for a manufacturer.or a supplier, a purchaser of the latex particle cannot participate. Under the circumstances, several methods in which a stabilizer such as a detergent or polysaccharides coexists in a storage liquid containing the latex particle carrying the antibody (or antigen) have been proposed. However, no method which can resolve all of the above problems has been found.

With the aim of solving the aforementioned problems in the latex aggregation reaction, the present inventors have conducted intensive studies and, as a result, successfully provided a method in which the antibody (or antigen) against the substance to be analyzed is not directly carried on the latex particle, and the degree of aggregation between latex particles can be analyzed.

The present invention is based on the above findings.

### DISCLOSURE OF INVENTION

The present invention relates to a latex aggregation method comprising the steps of:
(1) bringing a sample possibly containing a substance to be analyzed into contact with a biotin-conjugated antigen or antibody against said substance to be analyzed, and then bringing the whole into contact with an avidin-conjugated latex particle, said biotin-conjugated antigen or antibody containing one biotin molecule; and
(2) analyzing said substance to be analyzed by detecting the degree of aggregation between said avidin-conjugated latex particle, and an immunocomplex formed from said substance to be analyzed and said biotin-conjugated antigen or antibody.

Disclose herein is an immunological analyzing reagent comprising
(1) a composition comprising a biotin-conjugated antigen or antibody against a substance to be analyzed (hereinafter sometimes referred to as the first composition), and
(2) a composition comprising an avidin-conjugated microparticle (hereinafter sometimes referred to as the second composition),
wherein the biotin-conjugated antigen or antibody contains an amount of biotin which does not substantially agglutinate with the avidin-conjugated microparticle in the absence of the substance to be analyzed.

In the immunological analyzing reagent described herein, one biotin molecule conjugates to the antigen or antibody in the biotin-conjugated antigen or antibody contained in the first composition.

In the immunological analyzing reagent described herein, the avidin-conjugated microparticle contained in the second composition is an avidin-conjugated latex particle.

In the immunological analyzing reagent described herein, one biotin molecule conjugates to the antigen or antibody in the biotin-conjugated antigen or antibody.

In another preferred embodiment of the immunological analyzing method according to the present invention the sample possibly containing the substance to be analyzed is brought into contact with the biotin-conjugated antigen or antibody against the substance to be analyzed, and then the whole is brought into contact with the avidin-conjugated microparticle.

### BRIEF DESCRIPTION OF DRAWINGS

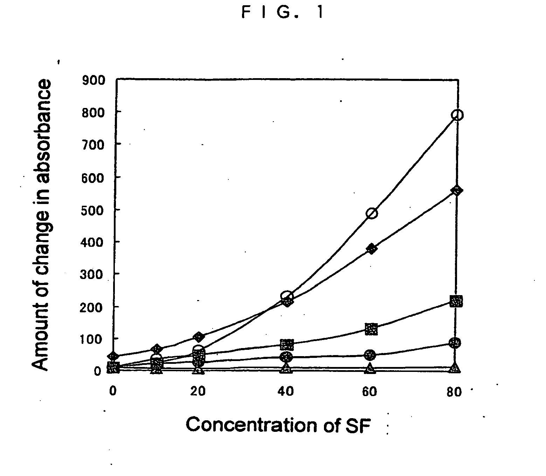

Figure 1 is a graph showing the results obtained by comparing a change of absorbance in the case when the analytical reagent which consists of two components according to the present invention prepared in Example 1 was used, to that in the case when the latex sensitized with the SF antibody prepared in Comparative Examples 1 and 2 was used.

### BEST MODE FOR CARRYING OUT THE INVENTION

The conjugates used in the method of the present invention are as described in claim 1. The conjugate contained in the first composition is a conjugate in which a predetermined amount of biotin conjugates to an antigen or antibody against a substance to be analyzed. A biotin-conjugated antigen or antibody containing an amount of biotin which does not substantially agglutinate with the avidin-conjugated microparticle in the absence of a substance to be analyzed (preferably one biotin molecule) is used as the conjugate.

For example, a conjugate in which two biotin molecules are conjugated to an antigen or antibody against a substance to be analyzed is prepared as the conjugate. When the conjugate and an avidin-conjugated microparticle coexist, a complex in which the avidin-conjugated microparticle, the conjugate, and another avidin-conjugated microparticle are bound in this sequential order will be formed, and then self-aggregation will occur, because avidin can specifically bind to biotin unless the substance to be analyzed coexists.

The term "an amount of biotin which does not substantially agglutinate with the avidin-conjugated microparticle in the absence of a substance to be analyzed" as used herein means an amount of biotin in which, when the avidin-conjugated microparticle and the biotin-conjugated antigen or antibody against a substance to be analyzed coexist in the absence of the substance to be analyzed, self-aggregation does not substantially occur. In this connection, the term "not substantially agglutinate" means that the results obtained by carrying out the analyzing method are not affected.

As previously described, the conjugate in which two biotin molecules are conjugated to an antigen or antibody causes self-aggregation. Therefore, the amount of biotin which may be conjugated to the antigen or antibody in the conjugate is not more than two molecules. The amount is preferably one molecule.

The conjugate may be prepared in accordance with a known method. For example, it may be obtained by chemically binding one or more biotin molecules (preferably one molecule) to the antigen or antibody by the use of a biotinylating agent or, alternatively, by physically binding one or more biotin molecules (preferably one molecule) to the antigen or antibody, removing the remaining biotin which does not adsorb or bind to the antigen or antibody, and diluting it with a buffer to an appropriate concentration. It can be judged whether or not the amount of biotin conjugated to the antigen or antibody is "an amount of biotin which does not substantially agglutinate with the avidin-conjugated microparticle in the absence of a substance to be analyzed", by analyzing whether or not self-aggregation substantially occurs when the resulting conjugate and the avidin-conjugated microparticle coexist in the absence of the substance to be analyzed.

The diluted liquid may be used as the first composition, without any modification, or by adding an appropriate additive such as a well-known stabilizer. Alternatively, the first composition may be prepared in powder form by lyophilizing the diluted liquid, without modification or after addition of an appropriate additive such as a well-known stabilizer. The stabilizer may be, for example, an inorganic salt, such as sodium chloride or sodium azide, a protein such as bovine serum albumin, or choline chloride.

The antigen or antibody which is conjugated to biotin in the conjugate is not particularly limited, so long as it can cause the antigen-antibody reaction with the substance to be analyzed. As the antibody including a monoclonal antibody and a polyclonal antibody, an immunoglobulin molecule per se or an antibody fragment [such as, Fab, Fab', F(ab')₂, or Fv] may be used. In this connection, when a monoclonal antibody is conjugated to biotin, two or more kinds of monoclonal antibodies which independently bind to the substance to be analyzed as an antigen at different sites, or one kind of monoclonal antibody having two or more recognition sites of the antigen may be used.

The avidin-conjugated microparticle contained in the second composition is a microparticle which is prepared by conjugating many avidin molecules to an inorganic or organic microparticle. Particles of organic high-molecular weight materials, for example, latex particles [i.e., microparticles of latex, such as polystyrene, styrene-methacrylic acid copolymer, styrene-glycidyl(meth)acrylate copolymer, or styrene-styrene sulfate copolymer] may be used as the microparticle.

An average particle size of the microparticle is not particularly limited, but is preferably 0.01 to 1.0 µm. When latex particles are used, the average particle size can be appropriately selected within the range of 0.05 to 1.0 µm (preferably 0.05 to 0.5 µm) in accordance with a measuring equipment used, or a detection concentration of the substance to be analyzed.

As the avidin, any avidin which can specifically bind to biotin, for example, an egg-white avidin, a streptoavidin, or an avidin prepared by a gene manipulation technique (i.e., a recombinant avidin) may be used.

The avidin-conjugated microparticle may be prepared, for example, by adding to microparticles (such as latex particles) a reagent for peptide synthesis (such as water-soluble carbodiimide) followed by avidin, stirring and centrifuging the whole, and dispersing the residue in water. The resulting avidin-conjugated microparticles (particularly avidin-conjugated latex particles) may be diluted with a buffer which can keep the dispersibility of the avidin-conjugated microparticles. The diluted liquid may be used as the second composition, without modification, or by adding an appropriate additive such as a well-known stabilizer. Alternatively, the second composition may be prepared in powder form by lyophilizing the diluted liquid, without modification or after addition of an appropriate additive such as a well-known stabilizer.

The buffer which can keep the dispersibility of the avidin-conjugated microparticles (particularly avidin-conjugated latex particles) may be prepared by adding a dispersing agent (such as TWEEN 20) to a conventional buffer [such as 10 mmol/L phosphate buffer (pH7.0)]. As the stabilizer for preparing the second composition, the stabilizer described for preparing the first composition may be used.

By the use of the first and second compositions prepared in the above-mentioned methods, for example, an assay kit which consists of two reagents, that is, the first reagent in liquid or powder form containing the conjugate and the second reagent in liquid or powder form containing the avidin-conjugated microparticle, may be prepared. In this connection, the composition in powder form is used after adding an appropriate buffer to produce a liquid form before use.

In the immunological analyzing method, a substance to be analyzed in a sample can be analyzed by bringing into contact (a) the sample possibly containing the substance to be analyzed, (b) a biotin-conjugated antigen or antibody against the substance to be analyzed, and (c) an avidin-conjugated microparticle, and detecting the degree of aggregation between the avidin-conjugated microparticle, and an immunocomplex formed from the substance to be analyzed and the biotin-conjugated antigen or antibody. In this case, the biotin-conjugated antigen or antibody contains an amount of biotin which does not substantially agglutinate with the avidin-conjugated microparticle in the absence of the substance to be analyzed (preferably one biotin molecule). The above-mentioned immunological analyzing reagent is suitable for carrying out the immunological analyzing method.

The contact between the sample (a), the conjugate (b), and the avidin-conjugated microparticle may be carried out at the same time. Alternatively, the sample (a) may be first brought into contact with the conjugate (b), and then the whole may be brought into contact with the avidin-conjugated microparticle (c). When the sample contains the substance to be analyzed, an immunocomplex is formed by the antigen-antibody reaction caused by the contact between the sample (a) and the conjugate (b), and a biotin-avidin complex is formed by the biotin-avidin reaction caused by the contact between the conjugate (b) and the avidin-conjugated microparticle (c). For example, an immunocomplex is formed by the antigen-antibody reaction caused by the contact between the sample (a) and the conjugate (b), and then biotin in the immunocomplex binds to avidin in the avidin-conjugated microparticle by the biotin-avidin reaction. The substance to be analyzed contained in the immunocomplex formed is brought into contact with another conjugate (b) to form another immunocomplex. The latter immunocomplex further binds to another avidin-conjugated microparticle, and then aggregation occurs. In addition, many avidin molecules exist in the avidin-conjugated microparticle and cause the similar biotin-avidin reaction and antigen-antibody reaction, and then the aggregation proceeds.

As a method for detecting the degree of aggregation, a known method such as a slide aggregation assay or an aggregation assay on a microplate may be used. When the degree of aggregation of latex particles is optically detected, for example, a known optical equipment for measuring an intensity of scattered light, absorbance, or an intensity of transmitted light may be used. The preferable measuring wavelength is 300 to 800 nm.

The method for detecting the degree of aggregation may be carried out in accordance with a known method, for example, by selecting a size of latex particle used, a latex concentration, a reaction time of the antigen-antibody reaction, and/or a reaction time of the biotin-avidin reaction, and measuring an increase or decrease, or the combination thereof, in an intensity of scattered light, absorbance, or an intensity of transmitted light.

In the immunological analyzing method, a concentration of the antibody or antigen contained in the antigen-antibody reaction system, an amount of biotin contained in the conjugate, or an amount of the avidin-conjugated microparticle may be appropriately selected in accordance with the sample or the substance to be analyzed. For example, the measuring range of an IgG (as the substance to be analyzed) concentration in serum (as the sample) is up to 50 mg/mL, and thus the amount used may be selected within the measuring range. Similarly, the measuring range of an elastase 1 (as the substance to be analyzed) concentration is up to 50 ng/mL, and thus the amount used may be selected within the measuring range.

In the immunological analyzing method, the conditions of the antigen-antibody reaction between the sample and the conjugate are the same as those of the conventional immunological analyzing method. In the immunological analyzing method, it is necessary that the conditions be suitable for the biotin-avidin reaction, because the biotin-avidin reaction between the conjugate and the avidin-conjugated microparticle is carried out in the same reaction system as that of the antigen-antibody reaction.

As a reaction medium for the antigen-antibody reaction and the biotin-avidin reaction, an appropriate buffer may be selected in accordance with the substance to be analyzed. As such a buffer, one having an appropriate ion concentration and pH which does not inactivate the substance to be analyzed and does not inhibit the antigen-antibody reaction and the biotin-avidin reaction may be used. For example, a Good's buffer, a glycine buffer, or a tris buffer may be used. The pH in the antigen-antibody reaction and the biotin-avidin reaction is preferably 5 to 10, more preferably 6 to 8. The reaction temperature is preferably 0 to 50°C, more preferably 20 to 40°C. The reaction time may be appropriately selected.

The sample which may be analyzed by the analyzing method of the present invention is not particularly limited, so long as it is a sample possibly containing an antigen or antibody as the substance to be analyzed. As the sample, there may be mentioned, for example, a liquid taken from a living body and generally used in a clinical diagnosis, such as blood, serum, plasma, or urine, or an experimental sample.

The substance to be analyzed in the analyzing method of the present invention is not particularly limited, so long as it is a substance (particularly a physiologically active substance) which may be generally analyzed by the use of the antigen-antibody reaction. As the substance to be analyzed, there may be mentioned, for example, proteins or lipids, such as antigens, antibodies, receptors, or enzymes. More particularly, there may be mentioned, for example, C-reactive protein (CRP), rheumatoid factor, ferritin, β-2 microglobulin, α-fetoprotein (AFP), an anti-streptolysin O antibody, IgE, a Treponema pallidum antibody, an anticardiolipin antibody, Hepatitis B virus (an HBS antibody, an HBS antigen, an HBc antibody, and an HBe antibody), D-dimer, fibrin(ogen) degradation products (FDP), Soluble fibrin (SF), plasmin-α2-plasmin inhibitor complex (PPI), a prostate-specific antigen (PSA), elastase 1, elastase XDP, thrombomodulin, or an anti-DNA antibody.

### EXAMPLES

The present invention now will be further illustrated by, but is by no means limited to, the following Examples.

### Example 1: Measurement of soluble fibrin

### (1) Preparation of a soluble fibrin antibody-conjugated biotin solution

As a monoclonal antibody against soluble fibrin (SF), monoclonal antibody FM No. 43-1 secreted by hybridoma FM No. 43-1 described in WO95/12617 was used. The hybridoma was domestically deposited in the International Patent Organism Depositary National Institute of Advanced Industrial Science and Technology [(Former Name) National Institute of Bioscience and Human-Technology Agency of Industrial Science and Technology (Address: AIST Tsukuba Central 6, 1-1, Higashi 1-chome Tukuba-shi, Ibaraki-ken 305-8566 Japan)] on October 27, 1993, and was transferred to an international deposit on October 27, 1994. The international deposit number (a number in parenthesis [] following the international deposit number is a domestic deposit number) is FERM BP-4846 [FERM P-13925].

More particularly, the following procedure was carried out in accordance with the method described in Example 2(b) of WO95/12617. An ascite containing the monoclonal antibody against soluble fibrin (SF) was prepared. A crude fraction purified from the ascite by the use of ammonium sulfate was treated with an ion exchange resin (DEAE) followed by a pepsin digestion to obtain F(ab')₂. To the resulting F(ab')₂, 2-mercaptoethyl amine was added to obtain an antibody fragment Fab'. The resulting antibody was dissolved in a 50 mmol/L phosphate buffer (pH7.1) at a concentration of 2.5 mg/mL, 0.6 mg of maleimide biotin (manufactured by Funakoshi Co., Ltd.) was added to the solution, and sensitization was carried out at room temperature for a day. A conjugate of the Fab' antibody and biotin was collected by a gel filtration on Superdex G-25 Superfine, and then the conjugate was added to a 0.1 mol/L phosphate buffer (pH6.5) at a concentration of 0.05 mg/mL to prepare an SF antibody-conjugated biotin solution.

### (2) Preparation of an avidin latex suspension

Water-soluble carbodimide (WSC) was added to 0.1 mL of 10% latex particle (particle size: 0.3 µm) at a final concentration of 2.5 mg/mL, and the whole was allowed to stand for 10 minutes. After 0.8 mg/mL of streptoavidin was added, the whole was stirred for an hour. The mixture was centrifuged, and 0.5 mL of distilled water was added to the resulting precipitate. The whole was stirred to obtain a 2% avidin latex dispersion. The resulting dispersion was diluted with a 10 mmol/L MOPS suspension (pH7.0) to prepare a 0.5% avidin latex suspension.

### (3) Reagent for analyzing an SF antigen

A reagent for analyzing human SF antigen in the present example is an assay kit which consists of two reagents, that is, the first reagent consisting of the SF antibody-conjugated biotin solution prepared in the above item (1) and the second reagent consisting of the avidin latex suspension prepared in the above item (2).

### (4) Standard SF antigen liquid

A clot obtained by reacting a commercially available fibrinogen (XIII factor free: manufactured by Kabi Diagnostica AB) with thrombin was solubilized with acetic acid, and added to plasma to prepare an SF antigen. Human plasmas containing the antigen at a concentration of 0 µg/mL, 10 µg/mL, 20 µg/mL, 40µg/mL, 60µg/mL, or 80µg/mL were used.

### (5) Analyzing method

Then 130 µL of the SF antibody-conjugated biotin solution prepared in the above item (1) was mixed with 3 µL of the standard SF antigen liquid prepared in the above item (4), and the whole was allowed to stand at 37°C for 5 minutes. After 130 µL of the avidin latex suspension prepared in the above item (2) was added, the whole was stirred. From the addition, absorbance at the wavelength of 600 nm was measured for 10 minutes. An amount of change in absorbance (ΔAbs) was the amount of change in the absorbance for 10 minutes. The measurement was carried out by the use of an automatic analyzer LPIA-S500. The result is shown in Fig. 1.

### Comparative Example 1

### (1) Physical adsorption of antibody to latex

The F(ab')₂ fragment of the anti-SF monoclonal antibody prepared in Example 1(1) was dissolved at a concentration of 0.5 mg/mL in a 10 mmol/L Tris-HCl buffer (pH8.0) to prepare an antibody liquid. To 5 mL of the antibody liquid, 5 mL of polystyrene latex (solid content = 5% (w/v), average particle size = 0.3 µm; JSR Corporation) was added, and the mixture was stirred at room temperature for 30 minutes.

To the mixture, a 100 mmol/L Tris-HCl buffer (pH8.0) containing 0.3% (w/v) bovine serum albumin (BSA) was added. The whole was stirred at room temperature for 60 minutes, and then centrifuged at 20,000 rpm.

To the resulting precipitate, a 10 mL of 10 mmol/L Tris-HCl buffer (pH8.0) containing 0.05% NaN₃ was added. The whole was stirred to prepare a latex reagent for comparison sensitized with the anti-SF antibody.

### (2) Measurement of SF

Then 130 µL of 0.1 mol/L phosphate buffer (pH6.5) was mixed with 3 µL of the standard SF antigen liquid prepared in Example 1(4), and the whole was allowed to stand at 37°C for 5 minutes. After 130 µL of the latex reagent sensitized with the anti-SF antibody prepared in Comparative Example 1(1) was added, the whole was stirred. From the addition, absorbance at the wavelength of 600 nm was measured for 10 minutes. The result is shown in Fig. 1.

### Comparative Example 2

(1) The SF antibody-conjugated biotin solution prepared in Example 1(1) was dissolved at a concentration of 0.5 mg/mL in a 10 mmol/L MOPS buffer (pH7.0). To the solution, the avidin latex used in Example 1(2) was added at a concentration of 1%. The whole was stirred at room temperature for 30 minutes to prepare SF antibody-sensitized latex. The resulting SF antibody-sensitized latex was diluted with 10 mmol/L MOPS suspension (pH7.0) to prepare 0.5% SF antibody-sensitized latex suspension, as a latex reagent for comparison sensitized with the anti-SF antibody.
(2) Then 130 µL of 0.1 mol/L phosphate buffer (pH6.5) was mixed with 3 µL of the standard SF antigen liquid prepared in Example 1(4). After 5 minutes, 130 µL of the latex reagent sensitized with the anti-SF antibody prepared in Comparative Example 2(1) was added, and the whole then stirred. From the addition, absorbance at the wavelength of 600 nm was measured for 10 minutes. The result is shown in Fig. 1.

In Fig. 1, the symbol ■ shows the result of the latex reagent sensitized with the anti-SF antibody prepared in Comparative Example 1, and the symbol ◆ shows the result of the latex reagent sensitized with the anti-SF antibody prepared in Comparative Example 2. The symbols ● and o show the results of the assay kit which consists of two reagents of the present invention in Example 1(5). The symbol • shows the result in which the concentration of the SF antibody-conjugated biotin was 0.0250 mg/mL, and the symbol o shows the result in which the concentration of the SF antibody-conjugated biotin was 0.05 mg/mL. The symbol A shows the result in the absence of the SF antibody-conjugated biotin (i.e., concentration of the SF antibody-conjugated biotin = 0.00 mg/mL) in Example 1(5).

As apparent from Fig. 1, according to the analyzing reagent, which is an assay kit which consists of two reagents, that is, the first reagent consisting of the SF antibody-conjugated biotin solution and the second reagent consisting of the avidin latex suspension, an excellent reactivity the same as or superior to that obtained by the conventional latex method (Comparative Example 1) was obtained. Further, according to the analyzing reagent, self-aggregation did not occur, and thus an amount of change in absorbance in the absence of the SF antigen (i.e., concentration = 0) was low, in comparison with the method in which the biotinylated antibody was previously bound to the avidin latex (Comparative Example 2). This result is very important for improving a measurement sensitivity.

### INDUSTRIAL APPLICABILITY

According to the present invention, a complicated procedure in which an antibody or antigen is carried on a microparticle such as a latex particle is not necessary. Further, self-aggregation of latex particles does not occur, and thus a storage stability is improved. Further, unlike the prior art, when an antibody or antigen against a substance to be analyzed is carried on latex, inactivation of the protein by adsorption to the latex surface does not occur. Therefore, the reactivity against the substance to be analyzed is not affected, and thus a precise measurement of the substance to be analyzed may be carried out. Further, in the measurement of the substance to be analyzed on the basis of the immunological aggregation reaction, the values of absorbance were increased and the sensitivity improved, in comparison with the method using a conventional antibody (or antigen)- sensitized latex (a method of immobilizaiton). Furthermore, according to the present invention, in comparison with the method in which the biotinylated antibody is previously bound to the avidin latex, the blank value becomes low, and thus the accuracy is improved, when the subject has a lower concentration of measuring object, and an excellent reactivity may be obtained, when the subject has a higher concentration of measuring object. The analyzing reagent of the present invention is suitable for use as an analyzing reagent for an automatic analyzing equipment.

As above, the present invention is explained with reference to particular embodiments, but modifications and improvements obvious to those skilled in the art are included in the scope of the present invention.

## Claims

1. A latex aggregation method comprising the steps of:
(1) bringing a sample possibly containing a substance to be analyzed into contact with a biotin-conjugated antigen or antibody against said substance to be analyzed, and then bringing the whole into contact with an avidin-conjugated latex particle, said biotin-conjugated antigen or antibody containing one biotin molecule; and
(2) analyzing said substance to be analyzed by detecting the degree of aggregation between said avidin-conjugated latex particle, and an immunocomplex formed from said substance to be analyzed and said biotin-conjugated antigen or antibody.

## Patentansprüche

1. Latex-Aggregations-Verfahren, das die folgenden Schritte umfasst:
(i) Inkontaktbringen einer Probe, die möglicherweise den zu analysierenden Stoff enthält, mit einem Biotin-konjugierten Antigen oder Antikörper gegen den Stoff und dann Inkontaktbringen des Ganzen mit einem Avidin-konjugierten Latex-Partikel, wobei der Biotin-konjugierte Antigen oder Antikörper ein Biotinmolekül enthält; und
(ii) Analysieren des zu analysierenden Stoffes durch Bestimmung des Aggregationsgrades zwischen dem Avidin-konjugierten Latex-Partikel und einem Immunkomplex, der sich zwischen dem zu analysierenden Stoff und dem Biotin-konjugierten Antigen oder Antikörper gebildet hat.

## Revendications

1. Une méthode d'agrégation du latex comprenant les étapes suivantes :
(1) mettre en contact un échantillon contenant éventuellement une substance à analyser avec un antigène conjugué à la biotine ou un anticorps contre ladite substance à analyser, et ensuite mettre en contacte l'ensemble obtenu avec des particules de latex conjuguées à l'avidine, ledit antigène conjugué à la biotine ou ledit anticorps contenant une molécule de biotine ; et
(2) analyser ladite substance à analyser par détection du degré de l'agrégation entre lesdites particules du latex conjuguées à l'avidine et un immunocomplexe formé par ladite substance à analyser et ledit antigène conjugué à la biotine ou ledit anticorps.
